# EUROPÄISCHE PATENTANMELDUNG

(11) **EP 2 520 642 A1**
(43) Veröffentlichungstag der Anmeldung: **07.11.2012**
(21) Anmeldenummer: 12166475.9
(22) Anmeldetag: 02.05.2012
(51) Int. Cl.: C12M 1/00

(54) **Photobioreaktor mit rotatorisch oszillierenden Lichtquellen**

(30) Priorität: 03.05.2011 DE 102011075110
(71) Anmelder: Bayer Intellectual Property GmbH, 40789 Monheim (DE)
(72) Erfinder: Brod, Helmut, 51061 Köln (DE); Frahm, Björn, 32657 Lemgo (DE)

(57) **Zusammenfassung**

Die Erfindung betrifft einen Photobioreaktor mit Belichtung durch rotatorisch oszillierend bewegte Lichtquellen (Lichtleiter, LEDs) sowie optional kombiniert rotatorisch oszillierend bewegte Membranflächen zum Gastransport.

Vorteile sind unter anderem ein im zeitlichen Mittel räumlich homogener Lichteintrag, welcher über Intensität und Oszillation an die Kultur und deren Dichte angepasst werden kann, sowie ein scherarmer Leistungseintrag und optional eine scherarme blasenfreie Be- und Entgasung.

## Beschreibung

Die vorliegende Erfindung betrifft einen Photobioreaktor, der rotatorisch oszillierend bewegbare Lichtquellen innerhalb des Reaktors für einen räumlich homogenen Lichteintrag aufweist.

Die Erfindung kann zur Züchtung von phototrophen Organismen genutzt werden, welche durch die rotatorisch oszillierend bewegbaren Lichtquellen innerhalb des Reaktors optimiert bestrahlt werden. Dieser optimierte Lichteintrag kann optional kombiniert werden mit einer optimierten Be- und Entgasung für scherempfindliche Reaktorinhaltsstoffe wie scherempfindliche phototrophe Organismen.

Ein bedeutendes Anwendungsgebiet von phototrophen Organismen ist die Züchtung von Algen. Neben der traditionellen Züchtung von Algen in flachen, sonnenbeschienenen Behältnissen wird zunehmend die Kultivierung von Algen in Bioreaktoren beschrieben. Eine Ursache für diese Entwicklung ist der Einsatz von Algen für die Herstellung von deutlich hochpreisigen Produkten. Dabei ergeben sich jedoch weitaus komplexere Anforderungen an die Züchtungsbedingungen bzw. an die Prozessführung. Dies kann nur in speziell konstruierten Photobioreaktoren realisiert werden. Die wirtschaftliche Realisierbarkeit der sich daraus ergebenen höheren Anschaffungs- und Produktionskosten von Photobioreaktoren im Vergleich zu den kostengünstigen traditionellen Züchtungsbehältnissen ergibt sich aus dem erzielbaren hohen Preisniveau der Produkte.

Derartige hochpreisige Produkte sind z.B. Farbstoffe in der Kosmetikindustrie oder mehrfach ungesättigte Fettsäuren wie natürliche Omega-3-Fettsäure oder Eicosapentaensäure (EPA) in der Nahrungsergänzungsmittelindustrie. In der Entwicklung der pharmazeutischen Industrie befinden sich Proteine für therapeutische und diagnostische Pharmazeutika. Ein weiteres Gebiet ist die grüne Energieproduktion (z. B. Produktion von Wasserstoff durch die Algen). Die Algenbiomasse, welche bei der Herstellung von Produkten aus Algen sowieso anfällt, kann außerdem bei der Produktion von Biogas verwendet werden, was diese Technologie besonders umweltfreundlich macht. Ein weiteres Beispiel für die Kombination von Algenzüchtung und Nutzung der Algenbiomasse zur Biogasproduktion ist die Rauchgasreinigung, bei der das enthaltene Kohlendioxid und der Kohlenstoff von den Algen verstoffwechselt wird. Somit kann der umwelttechnische Vorgang der Rauchgasreinigung mit dem energietechnischen Aspekt der Biogasgewinnung aus hergestellter Algenbiomasse symbiotisch verknüpft werden.

Im Stand der Technik werden Photobioreaktoren unterschiedlicher Bauweise beschrieben.

Havel et al. beschreibt einen Bioreaktor mit bis zu 16 parallel betriebenen Blasensäulen, die mit einer künstlichen Lichtquelle direkt über den Blasensäulen ausgerüstet sind, so dass ausreichende Lichtversorgung über ein homogenes Lichtspektrum gewährleistet wird. Um jede Blasensäule herum leiten zylindrische, reflektierende Rohre das Licht von oben zu den zylindrischen Glasaußenwänden jeder Blasensäule weiter, um die Lichtstreuung zu verringern und die Bestrahlung zu verbessern. Als Lichtquelle wird eine Kombination von fluoreszierenden Röhren von Osram Fluora L77 (Osram, Munich, Germany) und von Sun-Glo (Hagen, Holm, Germany) zur Erzeugung eines künstlichen Sonnenlichts verwendet [J. Havel, E. Franco-Lara, D. Weuster-Botz: "A parallel bubble column system for the cultivation of phototrophic microorganisms" Biotechnology Letters (2008) 30:1197-1200]. Innerhalb der Blasensäule kontrolliert die Strömungsdynamik die Lichtgeschichte der schwebenden photosynthetischen Zellen, wobei die Organismen sich quasi chaotisch in der Blasensäule bewegen. Für eine optimale Leistung müssen außerdem die Blasensäulen bei der höchst möglichen Begasungsrate gefahren werden, welche sich aus der Schertoleranz der Algen ergibt. Gleichzeitig darf die Begasungsrate nicht so hoch sein, dass ein Gasstau stattfinden kann, der die Lichtübertragung durch die Blasensäule verhindern würde [J. C. Merchuk, F. Garcia-Camacho, E. Molina-Grima "Photobioreactor Design and Fluid Dynamics" Chem. Biochemical Engineering Quarterly (2007) 21 (4):345-355]. Solche Photobioreaktoren werden trotzdem am meisten verwendet, weil sie einfach zu bauen und in Betrieb zu halten sind. Allerdings sind eine Maßstabsvergrößerung und eine optimale Steuerung dieser Art von Photoreaktoren schwierig und dadurch deren Ertrag üblicherweise niedrig. Die Ursache hierfür ist auch darin begründet, dass der Lichteintrag bei diesen Photobioreaktoren über die Außenoberfläche des Reaktors erfolgt. Mit größerem Maßstab wird das Oberfläche-zu-Volumen-Verhältnis jedoch kleiner und dadurch der Lichteintrag pro Volumen niedriger, wodurch die Produktausbeute limitiert wird.

Ein Rohrphotobioreaktor besteht aus geradem oder gewundenem arrangiertem durchsichtigen Rohrmaterial, dessen Arrangement den maximalen Sonnen/Lichtstrahlungsempfang erreichen soll. Das phototrophe Züchtungsmaterial wird innerhalb der Röhre befördert. Hierfür werden Airlift-Zirkulatoren besonders häufig eingesetzt [J. C. Merchuk, F. Garcia-Camacho, E. Molina-Grima "Photobioreactor Design and Fluid Dynamics" Chem. Biochemical Engineering Quarterly (2007) 21 (4):345-355]. Merchuk et al. gibt außerdem einen Überblick des Standes der Technik zur Züchtung von photosynthetischen Zellen in Bioreaktoren. Es werden außer den oben genannten Blasensäulen-Reaktoren und Rohrreaktoren auch Dünnfilm-Bioreaktoren und Airlift-Reaktoren beschrieben. Der Airlift-Reaktor im Vergleich zur Blasensäule ermöglicht eine kontrollierte Strömung durch Kanäle. In dem Aufbau, bestehend aus konzentrischer Röhre, befindet sich die Lichtquelle an den Außenwänden und die Innenwände definieren den dunklen Bereich [J. C. Merchuk, F. Garcia-Camacho, E. Molina-Grima "Photobioreactor Design and Fluid Dynamics" Chem. Biochemical Engineering Quarterly (2007) 21 (4):345-355].

Rastre et al. beschreibt eine Methode zum rationalen Design von Großmaßstabreaktoren durch die Kombination von Modell-Bioreaktor und Pilot-Bioreaktor. Geschlossene Röhren- und Plattenbioreaktoren werden untersucht [R. R. Sastre, Z. Csögör, I. Perner-Nochta, P. Fleck-Schneider, C. Posten, Journal of Biotechnology (2007) 132:127-133].

US 20100144019 beschreibt einen Photobioreaktor zur Züchtung von Mikroalgen, in dessen Behälter eine Mehrzahl von Lichtquellen verschiedener Länge, insbesondere Photodioden in Kombination mit einem Lichtleiter, ein mechanisches Rührmittel zur Erzeugung von Strömungen und ein pneumatisches Mischsystem, das Blasen erzeugt, um die Mikroalgen einzususpendieren, eingebracht sind, wobei die Lichtquellen innerhalb des Behälters eintauchen. Flexible Lichtquellen sind nicht offenbart.

WO 2009069967 beschreibt einen Photobioreaktor zur Züchtung von Mikroalgen bestehend aus einer Mehrzahl von Lichtquellenflächen, z.B. einer flexiblen LED-Folie, in einem Behälter. Die Lichtquellenflächen weisen die Form einer flachen Platte oder eines Zylinders auf und sind mit regelmäßigem Abstand in das Innere des Reaktorbehälters zu seiner Teilung eingebaut. Die Kultur wird in den Reaktor wie in einem Rohrreaktor von einem Eingang bis zu einem Ausgang entlang der Lichtquellenflächen befördert, wobei diese im Behälter so platziert sind, dass der Weg zwischen Eingang und Ausgang möglichst lang ist.

In US 20100028977 A1 wird ein Photobioreaktor zur Züchtung von Algen beschrieben, bei dem Licht über Stäbe in die Kulturflüssigkeit eingetragen wird. Die beschriebene Anordnung der Stäbe erlaubt jedoch keinen räumlich homogenen Lichteintrag in den Photobioreaktor. Als weiterer Nachteil wird zwar eine Rotation der angeordneten Stäbe beschrieben, jedoch ohne Erzeugung einer Relativgeschwindigkeit zwischen Lichtquelle und Kulturflüssigkeit mit den phototrophen Organismen. Diese Relativgeschwindigkeit führt zu einer entsprechenden Durchmischung und dazu, dass immer andere phototrophe Organismen in die Nähe der Lichtquelle transportiert werden. Die Relativgeschwindigkeit führt also dazu, dass im zeitlichen Mittel alle phototrophen Organismen die gleiche Lichtdosis erhalten und es somit keine Organismen gibt, die aufgrund größerer Entfernung von der Lichtquelle eine im zeitlichen Mittel geringere Lichtdosis erhalten. Ferner fehlt in dem Patent US 20100028977 A1 ein Be- und Entgasungskonzept für den beschriebenen Photobioreaktor. Dies ist ein weiterer Nachteil, da neben dem Lichteintrag für Algen auch die Zufuhr von Kohlendioxid und die Abfuhr von Sauerstoff von Bedeutung ist.

US 20100028977 A1 weist also die Nachteile auf eines nicht räumlich homogenen Lichteintrags, einer nicht vorhandenen Relativgeschwindigkeit zwischen Lichtquelle und Kulturflüssigkeit und eines fehlenden Gastransferkonzeptes.

In allen oben aufgeführten Photoreaktoren befindet sich entweder die Lichtquelle außerhalb des Züchtungsbehälters und tritt mit dem gezüchteten Material nicht in Berührung. Entsprechend werden energiestarke Lichtquellen benötigt, um die nötige Strahlung und eine möglichst hohe Eindringtiefe der Lichtstrahlung zu erzeugen. Bei dieser direkten Bestrahlung besteht das Risiko einer Photoinhibition bei besonders empfindlichen Organismen. Oder als andere Möglichkeit wird das Licht von der Lichtquelle mit Lichtleitern in den Photobioreaktor geführt oder beide Möglichkeiten kombiniert.

Trotz enormem Interesse in den letzten Jahren ist eine wirtschaftlich zufriedenstellende Lösung für die Züchtung von phototrophen Zellen, insbesondere Algen und Cyanobakterien in Bioreaktoren, noch nicht verfügbar.

In WO 2007098850 und WO 2010034428 wird ein Verfahren zur blasenfreien Begasung von Flüssigkeiten, insbesondere von Zellkulturen, mit einem Gasaustausch über eine oder mehrere flexibel in dem zu be- und/oder entgasenden Medium eingetauchte Membranflächen wie Schläuche, Zylinder oder Module beschrieben, bei dem die Membranfläche eine beliebige, rotatorisch oszillierende Bewegung in dem Medium ausführt. Die rotatorisch oszillierende Bewegung ist dabei einfach zu realisieren über eine entsprechende Ansteuerung des Antriebsmotors. Vom konstruktionstechnischen und mechanischen Aspekt her gibt es keine Zusatzanforderungen. Durch eine gezielte Abänderung der Bewegung kann die Bewegung so optimiert werden, dass die Anströmung der Membranfläche optimal ist. Da der Stofftransportkoeffizient von der Anströmung der Membranfläche abhängt, ist eine Bewegung optimal, bei der die Membranfläche jeweils eine möglichst hohe Relativgeschwindigkeit zur Flüssigkeit aufweist. Ein weiterer Vorteil der rotatorisch oszillierenden Bewegung der Membranfläche ist die Tatsache, dass ein separates Rühr- oder Mischorgan zur Erzeugung einer Anströmung der Membranfläche entfällt. Ferner werden bei der rotatorisch oszillierenden Bewegung keine Strömstörer im Behältnis benötigt. Bei konventionellen Rührorganen werden üblicherweise Stromstörer eingesetzt, um eine Mitbewegung der Flüssigkeit mit dem Rührer zu vermeiden und ausreichend Turbulenz und Leistungseintrag bereitzustellen. Diese Vereinigung von Membranflächenbereitstellung und Erzeugung der Membranflächenanströmung in WO 2007098850 und WO 2010034428 vermeidet Zonen von lokal hohem Leistungseintrag und lokal hoher Scherbeanspruchung. Bei dem beschriebenen Bioreaktor erfolgt der Leistungseintrag räumlich gleichmäßig und dient unmittelbar der Anströmung der Membranfläche. Mit einer gezielten Flüssigkeitsbewegung an allen Stellen der Membranfläche wird ein hoher, definierter Stofftransport infolge der Bewegung der Membranflächen relativ zur Flüssigkeit erzeugt. Insgesamt wird ein besseres Verhältnis von Stofftransport und dem zur Erzeugung des Stofftransports erforderlichen mechanischen Leistungseintrag bzw. und der unvermeidbaren Scherbeanspruchung im Bioreaktor erzielt als bei herkömmlichen Verfahren und Vorrichtungen. Eine Anwendung des Bioreaktors zur Züchtung von phototrophen Zellen ist nicht beschrieben, eine Lichtquelle ist in der beschriebenen Vorrichtung auch nicht vorgesehen.

Die Kultivierung von phototrophen Organismen wie Algen in Photobioreaktoren erfordert im Gegensatz zur Züchtung von Bakterien, Hefen oder Säugetierzellen in Bioreaktoren zusätzlich zur Gasver- und Gasentsorgung den Eintrag von Lichtenergie. Aufgrund der geringen Eindringtiefe der Lichtstrahlung ist ein möglichst homogener Lichteintrag erforderlich, bei dem möglichst keine Bereiche im Bioreaktor existieren, in denen die Lichtquelle so weit entfernt ist, dass die Produktivität dadurch beeinträchtigt ist. Die exponentielle Dämpfung der Bestrahlungsstärke erzeugt drei Zonen mit verschiedenen Wachstumsverhältnissen:
- In der ersten Zone, die von der Lichtquelle bis zu dem Punkt reicht, bei dem die Lichtenergie den Energiebedarf der Alge für maximale Wachstumsrate deckt, hängt die Wachstumsrate dann hauptsächlich von Zellenart und Züchtungsmedium ab. Gegebenenfalls kann Photoinhibition in dieser Zone das maximale Wachstum in der Nähe der Lichtquelle hindern.
- Die zweite Zone endet an dem Punkt, bei dem die an den Zellen ankommende Lichtenergie gerade dem Energiebedarf für Aufrechterhaltungsstoffwechsel gleicht. In dieser Zone ist Licht der limitierende Faktor und die photosynthetische Wachstumsrate ist proportional zu der ankommenden Lichtintensität.
- Die dritte Zone ist der unterbeleuchtete Bereich, in dem das Wachstum negativ ist und Fouling stattfindet.

Das o. g. Zonensystem lässt die Strömungsdynamik innerhalb des Bioreaktors außer Betracht.

Es entsteht also für die Züchtung von phototrophen Organismen wie Algen in Photobioreaktoren die Aufgabe, nicht nur wie in Bioreaktoren üblich, eine hohe Gasversorgung und Gasentsorgung bei ausreichender Durchmischung und Vermeidung von Fouling und Aggregatbildung bereitzustellen, sondern gleichzeitig zusätzlich einen möglichst homogenen ausreichenden Lichteintrag zu realisieren. Ferner muss beim Photobioreaktordesign neben dem zusätzlichen anspruchsvollen Aspekt des Lichteintrags je nach phototrophem Organismus auch der Scherempfindlichkeit verstärkt Rechnung getragen werden.

Scherbeanspruchung entsteht unter anderem auch aufgrund von Blasenbegasung, so dass eine blasenfreie Begasung von Vorteil ist, besonders bei phototrophen Organismen, die eine blasenfreie Begasung sowieso bevorzugen. Somit erschwert sich bei scherempfindlichen und/oder blasenempfindlichen Organismen die oben beschriebene Aufgabe. In dem Fall der blasenfreien Begasung muss besonders auf die Vermeidung von Fouling und Aggregatbildung geachtet werden. Homogener Lichteintrag mit Gasversorgung und -entsorgung, ggf. in blasenfreier Art und Weise, ausreichende Durchmischung und Vermeidung von Fouling und Aggregatbildung bei gleichzeitig geringer Scherbeanspruchung müssen also bereit gestellt werden, wobei geringe Scherbeanspruchung häufig auch geringen Leistungseintrag und damit geringe Gasversorgung und -entsorgung sowie Durchmischung bedingt.

Darüber hinaus wird ein Photobioreaktor benötigt, der wirtschaftlich zufriedenstellend aufzubauen, zu betreiben und in Betrieb zu halten ist und möglichst flexibel an die Bedürfnisse der gezüchteten Organismen angepasst werden kann.

Die Anforderungen an Photobioreaktoren werden durch die bisher existierenden Systeme nur in einzelnen oder mehreren Kriterien, aber nicht in allen ausreichend bedient.

Überraschend wurde die oben genannte Aufgabe durch einen Photobioreaktor gekennzeichnet durch in einem Bioreaktorflüssigvolumen verteilte, beliebig rotatorisch oszillierend bewegbare Lichtquellen und ein Verfahren zur Verwendung des erfindungsgemäßen Photobioreaktors gelöst.

Dabei soll der Begriff Lichtquelle in diesem Kontext nicht zwangsläufig bedeuten, dass die Lichtquelle das Licht erzeugt, sondern der Begriff Lichtquelle wird auch im Sinne von Lichtaustritt verwendet. So kann z.B. das Licht außerhalb des Photobioreaktors erzeugt und per Glasfaserkabel in den Bioreaktor geleitet werden, wo es dort aus Lichtleitern austritt. Diese fungieren für den Photobioreaktor als Lichtquelle.

Die Erfindung ist nicht nur für phototrophe Organismen wie Algen oder Cyanobakterien geeignet, sondern auch für entsprechende andere Applikationen mit Lichteintrag und ggf. Begasung.

Der Lichteintrag (vorzugsweise in Flüssigkeiten) dient hier der Bereitstellung von Licht für die Photosynthese. Durch die Anbringung der Lichtquelle (z.B. sternförmig auf einem Rotor) wird in Verbindung mit der Bewegung in der Flüssigkeit das Problem der geringen Lichteindringtiefe gelöst. Üblicherweise werden die Lichtquellen an einem oder mehreren Trägern fixiert. Geeignete Träger sind Membranflächen wie Schläuche, Zylinder oder weitere Module aus bevorzugt bioprozesstauglichem Stahl oder Kunststoff.

Vorzugsweise werden die Lichtquellen räumlich annähernd homogen verteilt. Vorzugsweise werden diese Lichtquellen statistisch über das komplette Volumen des Photobioreaktors verteilt.

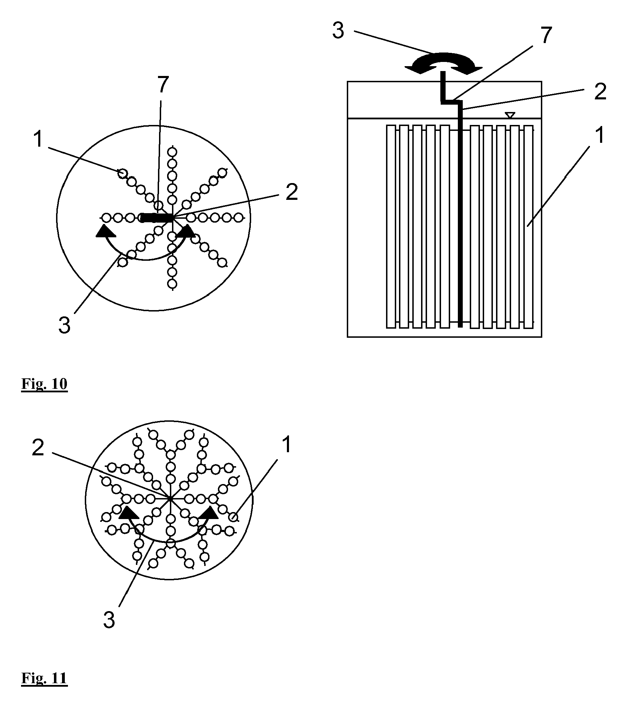

In einer bevorzugten Ausführungsform sind die Lichtquellen in Form von flexiblen Lichtleitern bzw. Leuchtdioden (LEDs) räumlich homogen im Photobioreaktor verteilt, z.B. durch Anordnung auf einem sternförmig verzweigten Rotor (siehe Beispiel in Fig. 11). Dadurch ist die Belichtung im Behältnis für den Fall einer nicht lichtabsorbierenden Behälterfüllung überall gleich. Eine vorteilhafte Anordnung der Lichtquellen zueinander im Verhältnis zu den Reaktorwänden und Reaktoreinbauten, kann auch mathematisch berechnet werden, um eine ideale Ausleuchtung zu erreichen. Der Rotor trägt z.B. am oberen und unteren Ende, also z.B. unter der Flüssigkeitsoberfläche und über dem Boden des Bioreaktors sternförmig Rotorarme. Die flexiblen Lichtleiter sind dann z.B. vertikal auf die Rotorarme gewickelt, z.B. vom oberen Rotorarm zum unteren, wieder zum oberen usw. Eine Riffelung der Rotorarme auf dem Auflagebereich kann das sichere Aufliegen der Lichtleiter unterstützen. Es sind bevorzugt dünne Lichtleiter zu verwenden, um deren Oberfläche-zu-Volumen-Verhältnis zu maximieren. Die Lichtquellen werden infolge der diskontinuierlichen Bewegung der Rotorwelle tangential angeströmt.

Die beschriebene Anordnung von Lichtquellen bzw. Lichtleitern, LEDs im Photoreaktor ist ideal für die Maßstabsvergrößerung (Scale-up) oder die Maßstabsverkleinerung (Scale-down) des Photobioreaktors, da das Verhältnis von Lichtquellenoberfläche bzw. Lichtleiteroberfläche zu Photobioreaktorvolumen konstant gehalten werden kann.

Durch die unmittelbare Nähe der Lichtquelle zu den Zellen sind verhältnismäßig energieschwache, d.h. auch energiesparende Lichtquellen geeignet.

Beispiele für Lichtleiter sind Lichtwellenleiter, Glasfasern, polymere optische Fasern oder andere lichtleitende Bauteile aus Kunststoff sowie Faseroptik-Komponenten.

### Daneben können Leuchtdioden (LEDs) als Lichtquellen im Reaktor verwendet werden.

Durch die gezielte Auswahl der Halbleitermaterialien und der Dotierung können die Eigenschaften des erzeugten Lichtes variiert werden. Vor allem der Spektralbereich und die Effizienz lassen sich so beeinflussen:
- Aluminiumgalliumarsenid (AlGaAs) - rot (665 nm) und infrarot bis 1000 nm Wellenlänge
- Galliumarsenidphosphid (GaAsP) und Aluminiumindiumgalliumphosphid (AlInGaP) - rot, orange und gelb
- Galliumphosphid (GaP) - grün
- Siliziumkarbid (SiC) - erste kommerzielle blaue LED; geringe Effizienz
- Zinkselenid (ZnSe) - blauer Emitter, der jedoch nie die kommerzielle Reife erreichte
- Indiumgalliumnitrid (InGaN)/Galliumnitrid (GaN) - ultraviolett, violett, blau und grün
- Weiße LEDs sind meistens blaue LEDs mit einer davor befindlichen Fluoreszenz-Schicht, die als Wellenlängen-Konverter wirkt

Die Anwendung von Lichtleitern und/oder Leuchtdioden zur Wachstumsbeförderung von phototrophen Organismen in einem erfindungsgemäßen Photobioreaktor ist ebenfalls Gegenstand der vorliegenden Erfindung.

In den rotatorisch oszillierenden Photobioreaktoren werden bevorzugt LEDs mit einer resultierenden Bestrahlungsstärke von etwa 5-120 µMol/m²·s, besonders bevorzugt etwa 5-30 µMol/m²·s eingesetzt.

Vorzugsweise werden die Lichtquellen mittels einer Lichtkontrolleinheit gesteuert. Insbesondere LEDs lassen sich über den Betriebsstrom sehr schnell schalten und modulieren. Dadurch kann der Lichteintrag (Lichtenergie, Lichtintensität) z.B. der Kulturdichte angepasst werden. Ferner ist eine Pulsation der Lichtquelle gut realisierbar.

Die Lichtquellen versorgen durch die rotatorisch oszillierende Bewegung alle phototrophen Organismen wie Algenzellen im zeitlichen Mittel möglichst gleichmäßig mit Licht. Die oszillierend rotatorische Bewegung erzeugt eine Relativgeschwindigkeit zwischen Lichtquelle und Reaktorinhalt und führt dazu, dass im zeitlichen Mittel jeder Teil des Reaktorinhaltes die gleiche Lichtdosis erhält. Die Erscheinung, dass z.B. bei sternförmiger Anordnung der Lichtquellen die Relativgeschwindigkeit zwischen Lichtquelle und Reaktorinhalt mit abnehmendem Abstand zur Oszillationsachse abnimmt, ist nicht von Bedeutung, da der Reaktorinhalt gut durchmischt ist und so ein Teilchen im Reaktor sich im Laufe der Zeit in unterschiedlichen Abständen zur Oszillationsachse aufhält.

Die mit der Oszillation einhergehende schwankende Lichtintensität, welche eine einzelne phototrophe Organismenzelle wie eine Algenzelle erfährt, ist unkritisch, da die Bestrahlung von Algen sowieso üblicherweise gepulst (z.B. mit einer Frequenz von einer Sekunde) erfolgt. Wichtig ist nur, dass wie bei dieser Erfindung alle Algenzellen im Mittel die gleiche ausreichende Lichtmenge erfahren. Somit wird dem Bedarf der Technik, phototrophe Organismen wie Algen in Reaktoren trotz der geringen Eindringtiefe der Lichtstrahlung kultivieren zu können, Rechnung getragen. Rühr- oder Mischorgane zur Anströmung der Lichtquellenfläche entfallen, wodurch Zonen von lokal hohem Leistungseintrag und lokal hoher Scherbeanspruchung vermieden werden. Dies trägt der Scherempfindlichkeit einiger phototropher Organismen (z.B. einiger Algenarten) Rechnung.

Die Oszillation kann außerdem angepasst werden, z.B. an die Kulturdichte. Eine höhere Kulturdichte, z.B. an Algen, führt zu einer geringeren Eindringtiefe des Lichts. Dies kann kompensiert werden durch eine Verstärkung der Oszillation bei höherer Kulturdichte, ggf. kombiniert mit einer Steigerung der Lichtenergie der Lichtquelle. Dadurch wird vermieden, dass die Beleuchtung pro Algenzelle bei steigender Kulturdichte abnimmt.

Der erfindungsgemäße Photobioreaktor ist verhältnismäßig einfach und kostengünstig bereitzustellen und kann an die Bedürfnisse der gezüchteten Organismen relativ unkompliziert angepasst werden.

Typischerweise ist der erfindungsgemäße Photobioreaktor zylindrisch, mit einer vorzugsweisen Größe von 1 L bis 1000 L Füllvolumen und mit Standard-Höhe-zu-Durchmesser-Verhältnissen, wodurch die Umrüstung vorhandener Bioreaktoren möglich ist.

Wird eine blasenfreien Begasung zum Transport von z. B. O₂ oder CO₂ benötigt, weist der erfindungsgemäße Photobioreaktor rotatorisch oszillierend bewegbare Mittel zu Gastransporten, ausgewählt aus der Gruppe enthaltend Sparger, Microsparger oder Membranflächen, insbesondere Membranschläuche auf. Die Begasungsmembranfläche kann zusätzlich zu den Lichtquellenträgern auf dem sogenannten Rotor installiert werden, z.B. in Form zusätzlicher Rotorarme analog wie in der eben geschilderten bevorzugten Ausführungsform. Alternativ können Begasungsmembranflächen, z.B. Begasungsschläuche, mit einer oder mehreren Lichtquellen ausgestattet sein.

Die Begasung von Flüssigkeiten dient dem Eintrag und der Desorption von Gasen. In der Biotechnologie sind weitere Ziele, mit einer entsprechenden Membranbegasung hohe Stofftransportkoeffizienten bei gleichzeitig geringem Leistungseintrag bzw. gleichzeitig geringer Scherbelastung zu erreichen. Der Gasaustausch erfolgt über eine oder mehrere beliebig geartete, eingetauchte Membranflächen, wobei die Membranfläche eine beliebige, rotatorisch oszillierende Bewegung in der Flüssigkeit ausführt. Die Membranbegasung, welche blasenfrei betrieben werden kann, trägt der Scherempfindlichkeit und dem Anspruch auf blasenfreie Begasung einiger phototropher Organismen, wie z.B. einiger Algenarten, Rechnung. Gegebenenfalls kann die Membranfläche auch so gewählt werden, dass diese nur die Gasversorgung und -entsorgung unterstützt. Die Membranfläche kann z.B. aus einem oder mehreren Membranschläuchen gebildet werden.

An der beliebig rotatorisch oszillierenden Lichtquelle können alternativ oder zusätzlich zu der Begasungsmembranfläche ein oder mehrere Sparger oder Microsparger angebracht werden, vorzugsweise am unteren Ende, wodurch die Blasenaufstiegsstrecke länger ist. Zudem steigen die Blasen oder Mikroblasen für den Gastransfer entlang der Lichtquelle auf, was zusätzlich zu einer entsprechenden Durchmischung der Bereiche und damit zu einem im zeitlichen Mittel gleichmäßigeren Lichteintrag führt, da die Durchmischung immer andere Organismen in die Nähe der Lichtquelle transportiert. In Verbindung mit der beliebig rotatorisch oszillierenden Bewegung werden dann alle Bereiche des Bioreaktors mit (Mikro)blasen versorgt.

Dieses Verfahren und diese Vorrichtung besitzen den weiteren Vorteil, dass die Agglomeratbildung und die Anlagerung von Substanzen an die Innenbereiche des entsprechenden Kultivierungsgefäßes/Photobioreaktors vermieden oder stark reduziert werden. Derartige Phänomene sind im Allgemeinen von Nachteil, da die Funktion von Elementen im Bioreaktor, wie z.B. Membranen zum Gastransfer oder Sonden, zum Teil erheblich eingeschränkt wird oder sogar aufgehoben wird [WO 2010034428]. Bei der Kultivierung von phototrophen Organismen wie Algen im erfindungsgemäßen Photobioreaktor wird ferner die Anlagerung von Substanzen an die Lichtquellen vermieden oder stark reduziert.

Ferner kann entsprechend den Kultivierungsanforderungen der jeweiligen Algen bzw. phototrophen Organismen nicht nur das Verhältnis von Lichtquellenfläche und Membranfläche zu Bioreaktorvolumen einfach variiert werden, sondern auch das Verhältnis von Lichtquellenfläche zu Membranfläche. In einer bevorzugten Ausführungsform können z.B. Membranschläuche und Lichtleiter auf Rotorarme aufgewickelt werden (z.B. alternierend), wobei die oben genannten Verhältnisse durch die Anzahl der Rotorarme und die Anzahl der Membranschläuche in Relation zu der Anzahl der Lichtleiter variiert werden können. Die Membranschläuche und Lichtleiter werden dabei vorzugsweise von einer außerhalb des Gefäßes angeordneten Gasversorgung und Lichtquelle versorgt.

In einer weiteren Ausführungsform der Erfindung verfügt der Photobioreaktor über Richtelemente, mit denen die Auslenkung der Membranfläche in einer Rotationsrichtung begrenzt werden kann, wobei die Richtelemente Lichtleiter oder Lichtdioden aufweisen können.

Bevorzugt sind außerdem die Lichtquelle, die Mittel zu Gastransporten oder beide über eine gemeinsame Halterung mit einer oder mehreren Sonden verbunden, die die Überwachung der Prozesse innerhalb des Reaktors ermöglichen.

Weiterer Gegenstand der vorliegenden Erfindung ist ein Verfahren zur Verwendung des erfindungsgemäßen Photobioreaktors, dadurch gekennzeichnet, dass die Lichtquellen eine beliebige diskontinuierliche Bewegung, bevorzugt eine beliebige Bewegung mit Bewegungsumkehr insbesondere Richtungsumkehr, besonders bevorzugt eine rotatorisch oszillierende Bewegung ausführen.

Ferner wird bevorzugt, dass die Mittel zu Gastransporten auch eine beliebige diskontinuierliche Bewegung, eine Bewegung mit Bewegungsumkehr-insbesondere Richtungsumkehr oder eine rotatorisch oszillierende Bewegung ausführen. In einer besonderen Ausführungsform des Verfahrens bewegen sich die Lichtquellen, die Mittel zu Gastransporten oder beide in einer periodischen Abfolge von Beschleunigung und Abbremsung zwischen zwei Bewegungsumkehrpunkten.

Das erfindungsgemäße Verfahren ist zur Kultivierung von Algen oder phototrophen Organismen besonders geeignet.

Weiterer Gegenstand der vorliegenden Erfindung ist die Verwendung von Lichtleitern, Leuchtdioden oder von beiden zur Wachstumsbeförderung von phototrophen Organismen in einem Photobioreaktor.

### Abbildungen

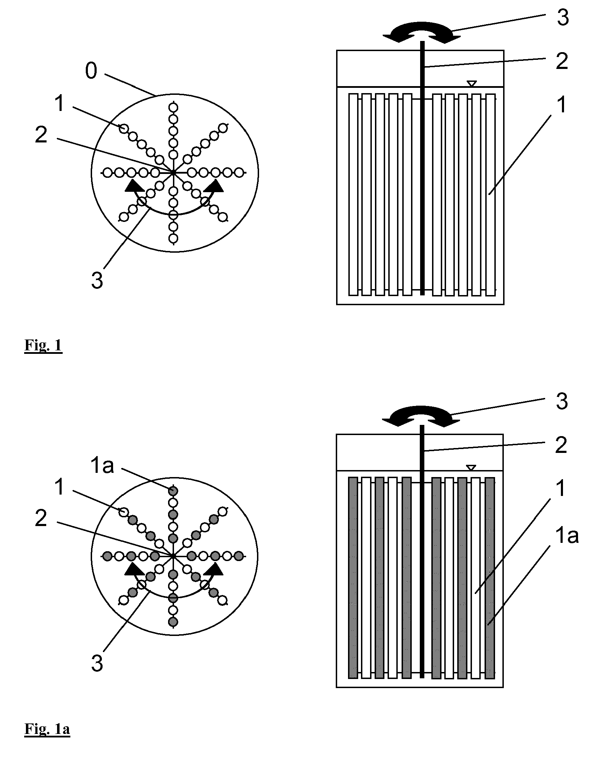

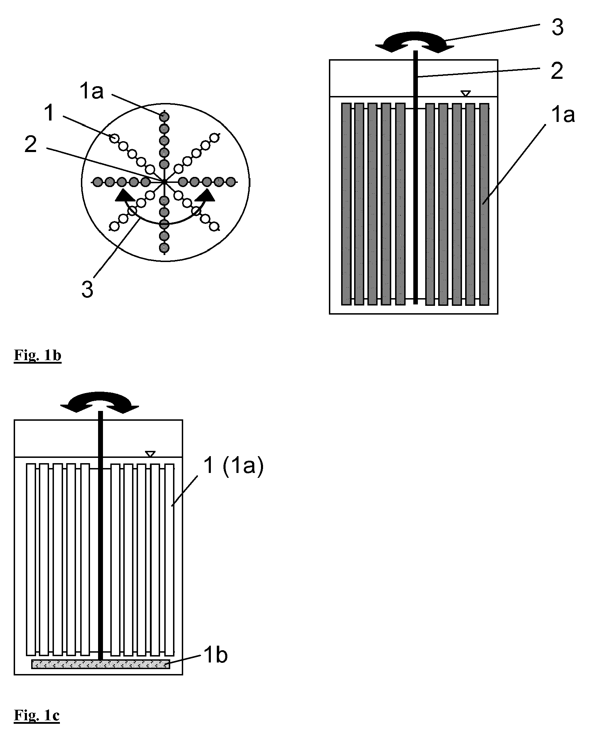

Figuren 1 bis 11 zeigen mögliche Ausführungsformen des erfindungsgemäßen Photobioreaktors ohne sich darauf zu begrenzen.
- Fig. 1:: Schematische Darstellung einer rotatorisch oszillierenden Bewegung zum Lichteintrag in einem Behältnis. Auf einen Rotor gewickelte Lichtleiter (1) bilden hier die Lichteintragsfläche. Diese rotiert mit der Rotorwelle (2) in beide Rotationsrichtungen (3).
- Fig. 1 a:: Schematische Darstellung einer rotatorisch oszillierenden Bewegung zum Lichteintrag in einem Behältnis kombiniert mit Einrichtungen zum Gastransfer. Zusätzlich zu den Lichtleitern sind hier Membranschläuche (1a) für den Gastransfer auf den Rotor gewickelt (Lichtleiter und Membranschlauch immer abwechselnd und bei jedem nächsten Rotorarm versetzt abwechselnd).
- Fig. 1b:: Schematische Darstellung einer rotatorisch oszillierenden Bewegung zum Lichteintrag in einem Behältnis kombiniert mit Einrichtungen zum Gastransfer. Zusätzlich zu den Lichtleitern sind hier Membranschläuche (1a) für den Gastransfer auf den Rotor gewickelt (Lichtleiter auf jedem zweiten Rotorarm und Membranschläuche auf allen übrigen Rotorarmen).
- Fig. 1c:: Schematische Darstellung einer rotatorisch oszillierenden Bewegung zum Lichteintrag in einem Behältnis kombiniert mit Einrichtungen zum Gastransfer. Zusätzlich zu den Lichtleitern ist hier ein Sparger (1b) (z.B. Microsparger) unterhalb der Lichtleiterflächen angeordnet.

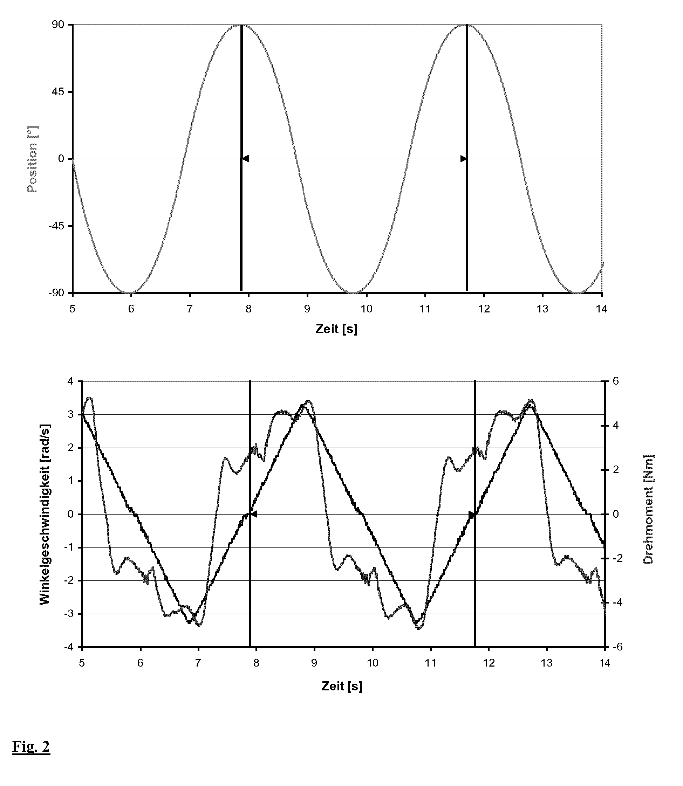

- Fig. 2:: Position, Winkelgeschwindigkeit und Drehmoment einer rotatorisch oszillierenden Bewegung zum Lichteintrag bzw. optional zur Be- und Entgasung von Flüssigkeiten.

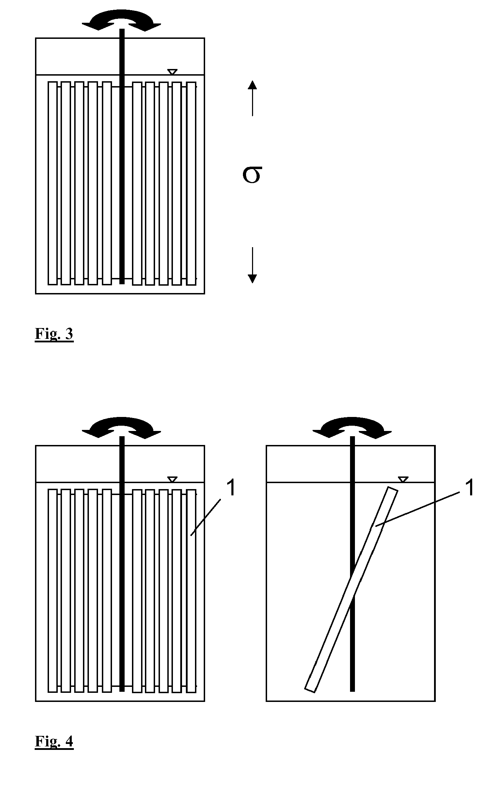

- Fig. 3:: Schematische Darstellung der Vorrichtung, gekennzeichnet durch eine Möglichkeit, die Spannung σ der Lichteintragsfläche, z.B. bestehend aus Lichtleitern und optional aus Membranschläuchen, zu variieren. Auf einen Rotor gewickelte Lichtleiter bilden hier die Lichteintragsfläche.
- Fig. 4:: Schematische Darstellung der Vorrichtung, gekennzeichnet durch eine Möglichkeit, den Anstellwinkel der Lichteintragsfläche zu variieren. Auf einen Rotor gewickelte Lichtleiter (1) bilden hier die Lichteintragsfläche.

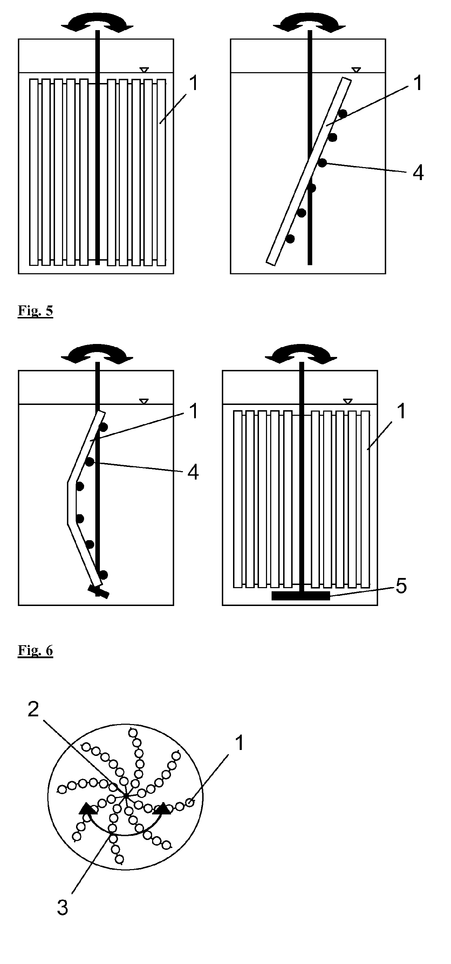

- Fig. 5:: Schematische Darstellung der Vorrichtung, gekennzeichnet durch eine Möglichkeit, die durch den Strömungswiderstand bedingte Auslenkung der Lichteintragsfläche durch Richtelemente (4) in einer Rotationsrichtung zu begrenzen. Auf einen Rotor gewickelte Lichtleiter (1) bilden hier die Lichteintragsfläche.
- Fig. 6:: Schematische Darstellung der Vorrichtung, gekennzeichnet durch eine Möglichkeit, die Lichteintragsfläche zur besseren Durchmischung durch Richtelemente (4) entsprechend zu formen und/oder auch Rührerblätter/Paddel (5) oder sonstige Vorrichtungen zur Strömungsführung und Durchmischung anzubringen. Auf einen Rotor gewickelte Lichtleiter (1) bilden hier die Lichteintragsfläche.

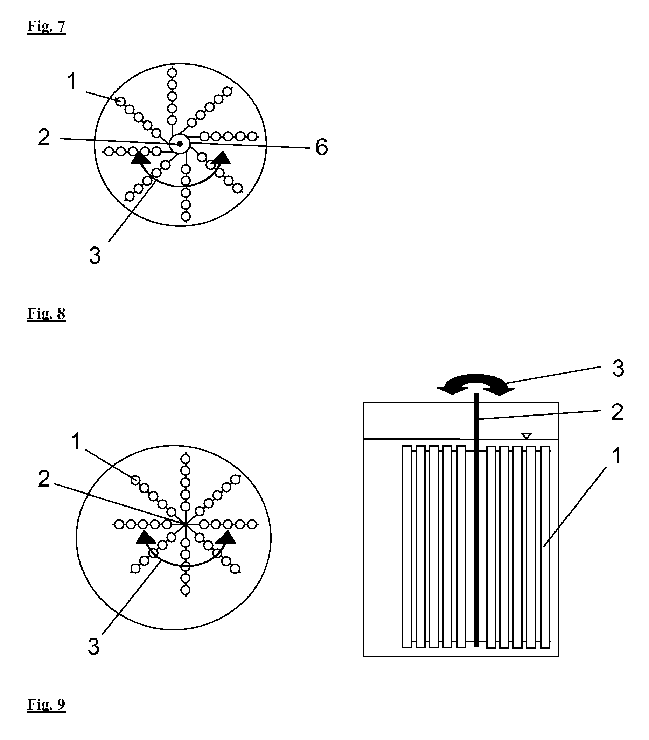

- Fig. 7:: Schematische Darstellung der Vorrichtung, gekennzeichnet durch eine Möglichkeit, die Durchmischung dadurch zu verbessern, dass die Rotorarme um die Rotorwelle (2) in einer der Rotationsrichtungen (3) gebogen ausgeführt werden. Auf einen Rotor gewickelte Lichtleiter (1) bilden hier die Lichteintragsfläche.
- Fig. 8:: Schematische Darstellung der Vorrichtung, gekennzeichnet durch eine Möglichkeit, die Durchmischung dadurch zu verbessern, dass die Rotorarme tangential um die Rotorwelle (2) in einer der Rotationsrichtungen (3) an einer Vorrichtung (6) angebracht werden. Auf einen Rotor gewickelte Lichtleiter (1) bilden hier die Lichteintragsfläche.
- Fig. 9:: Schematische Darstellung der Vorrichtung, gekennzeichnet durch eine Möglichkeit, die Durchmischung dadurch zu verbessern, dass die Rotorwelle (2) mit den beiden Rotationsrichtungen (3) exzentrisch im Behältnis angebracht wird. Auf einen Rotor gewickelte Lichtleiter (1) bilden hier die Lichteintragsfläche.
- Fig. 10:: Schematische Darstellung der Vorrichtung, gekennzeichnet durch eine Möglichkeit, die Durchmischung dadurch zu verbessern, dass die Rotorwelle (2) mit den beiden Rotationsrichtungen (3) zwar zentrisch im Behältnis angebracht wird, dann aber einen Exzenter (7) aufweist. Auf einen Rotor gewickelte Lichtleiter (1) bilden hier die Lichteintragsfläche.
- Fig. 11:: Schematische Darstellung der Vorrichtung, gekennzeichnet durch eine Möglichkeit, die Lichteintragsfläche pro Volumen möglichst gleichmäßig um die Rotorwelle (2) mit den beiden Rotationsrichtungen (3) zu verteilen. Auf einen Rotor gewickelte Lichtleiter (1) bilden hier die Lichteintragsfläche.

### Bezugszeichen:

0- Behältnis des Photobioreaktors
1- Einrichtung für Lichteintrag, z.B. Lichtleiter, Lichtquellen, LEDs
1a- Einrichtung für Gastransfer, z.B. Membranschläuche
1b- Einrichtung für Gastransfer, z.B. (Micro)Sparger
2- Rotorwelle
3- Rotationsrichtung
4- Richtelemente, mit denen die Auslenkung der Lichtleiter und ggf. der Membranschläuche in einer Rotationsrichtung begrenzt wird. Diese Richtelemente können Lichtleitern oder LEDs aufweisen.
5- Rührer
6- Vorrichtung zur tangentialen Anordnung der Rotorarme
7- Exzenter in Rotorwelle
σ- Spannung

### Beispiel:

Die Erfindung wird nachstehend anhand eines Beispiels näher erläutert, ohne sie jedoch auf dieses zu beschränken.

In Fig. 1 ist schematisch ein Beispiel einer Vorrichtung zur Ausführung des erfindungsgemäßen Verfahrens dargestellt. Die Lichtquelle wird durch Lichtleiter (1) gebildet, die an einer Rotorwelle (2) vertikal quer zur Rotationsrichtung (3) angeordnet sind. Durch die flexiblen Lichtleiter kann Licht von einer Lichtquelle, vorzugsweise außerhalb des Bioreaktors angeordnet, in das Innere geleitet werden, wo es gleichmäßig aus dem Lichtleiter austritt. Durch die ggf. ergänzenden Membranschläuche (1a in Fig. 1a und Fig. 1b) kann z.B. kohlendioxidhaltiges Gas zur Versorgung von Organismen wie Algen und zum Abtransport von gebildetem Sauerstoff geführt werden. Die Membranschläuche und Lichtleiter können immer abwechselnd und bei jedem nächsten Rotorarm versetzt abwechselnd aufgewickelt werden (Fig. 1a) oder es können z.B. Lichtleiter auf jedem zweiten Rotorarm und Membranschläuche auf allen übrigen Rotorarmen angebracht werden (Fig. 1b). Eine weitere Möglichkeit zum Gastransfer ist die Anbringung eines Spargers (1b) (z.B. Microsparger) unterhalb der Lichtleiterflächen (Fig. 1c).

Die Vorrichtung zum Lichteintrag wird bevorzugt innerhalb eines Photobioreaktors (0) betrieben und das erzeugte Licht über geschlossene Lichtleiter in den Photobioreaktor geleitet, wo das Licht dann aus der Oberfläche der auf die Rotorarme gewickelten Lichtleiter austritt. Die Lichtleiter sind mit geringem Abstand nebeneinander auf die Rotorarme zu wickeln. Die Oberfläche der Rotorarme ist zum geordneten Aufwickeln und zur Verhinderung des Verrutschens vorzugsweise mit Vertiefungen versehen.

Bevorzugt tauchen die Lichtleiter und ggf. die Membranschläuche vollständig in das Kulturmedium ein. Die Vorrichtung kann eine rotatorische Bewegung um die Rotorwelle (2) ausführen. Bevorzugt führt sie eine rotatorisch oszillierende Bewegung aus. Diese Bewegung führt zum einen zu einer verbesserten Versorgung der Organismen im Bioreaktor mit Licht und bei optionaler Verwendung von Membranschläuchen zu einem (blasenfreien) Gastransport und zum anderen zu einer deutlich verringerten Neigung zur Bildung von Ablagerungen und Agglomeraten (im Vergleich zu einer statischen Licht- und ggf. Membranfläche, die durch ein Rührwerk angeströmt wird).

Fig. 2 zeigt exemplarisch eine Oszillation des Rotors von je 180° in die eine Richtung und anschließend von 180° zurück in die Ausgangsstellung. Diese Oszillation findet mit einer konstanten Winkelgeschwindigkeit statt (Fig. 2). Die entstehende Relativgeschwindigkeit zwischen Rotor und Reaktorinhalt ist an dem aufgetragenen Drehmoment zu erkennen (Fig. 2).

Fig. 3 zeigt eine Möglichkeit, die Spannung σ der Lichteintragsfläche, z.B. bestehend aus Lichtleitern und optional aus Membranschläuchen, zu variieren.

Fig. 4-6 zeigen Möglichkeiten, den Anstellwinkel zu variieren (Fig. 4), die durch den Strömungswiderstand bedingte Auslenkung der Lichteintragsfläche durch Richtelemente (4) in einer Rotationsrichtung zu begrenzen (Fig. 5) und die Lichteintragsfläche zur besseren Durchmischung durch Richtelemente entsprechend zu formen und/oder auch Rührerblätter/Paddel (5) oder sonstige Vorrichtungen zur Strömungsführung und Durchmischung anzubringen (Fig. 6).

Fig. 7-10 zeigen Möglichkeiten, die Durchmischung zu verbessern - Rotorarme um die Rotorwelle in einer der Rotationsrichtungen gebogen auszuführen (Fig. 7), Rotorarme tangential um die Rotorwelle in einer der Rotationsrichtungen an einer Vorrichtung (6) anzubringen (Fig. 8), die Rotorwelle exzentrisch im Behältnis anzubringen (Fig. 9), die Rotorwelle zwar zentrisch im Behältnis, aber mit einem Exzenter (7) anzubringen (Fig. 10).

Fig. 11 zeigt eine Möglichkeit, die Lichteintragsfläche pro Volumen möglichst im Behältnis zu verteilen.

Vorzugsweise werden 8 Rotorarme verwendet, vorzugsweise mit Verzweigung der Rotorarme wie in Fig. 11 gezeigt.

## Patentansprüche

1. Photobioreaktor **gekennzeichnet durch** in einem Bioreaktorflüssigvolumen verteilte, beliebig rotatorisch oszillierend bewegbare Lichtquellen.

2. Photobioreaktor nach Anspruch 1, **dadurch gekennzeichnet, dass** die Lichtquellen räumlich annähernd homogen verteilt sind.

3. Photobioreaktor nach einem der Ansprüche 1 oder 2, **dadurch gekennzeichnet, dass** die Lichtquellen aus einem oder mehreren Lichtleitern oder Lichtdioden in einer oder mehreren Membranflächen gebildet sind.

4. Photobioreaktor nach einem der Ansprüche 1 bis 3, der über rotatorisch oszillierend bewegbare Mittel zu Gastransporten, ausgewählt aus der Gruppe enthaltend Sparger, Microsparger oder Membranflächen, verfügt.

5. Photobioreaktor nach einem der Ansprüche 3 bis 4, wobei die Membranfläche an sternförmig an einer Rotorwelle angebrachten Rotorarmen angebracht ist.

6. Photobioreaktor nach einem der Ansprüche 3 bis 5, umfassend Richtelemente, mit denen die Auslenkung der Membranfläche in einer Rotationsrichtung begrenzt werden kann.

7. Photobioreaktor nach Anspruch 5, wobei die Richtelemente Lichtleiter oder Lichtdioden aufweisen.

8. Photobioreaktor nach einem der Ansprüche 1 bis 7, **dadurch gekennzeichnet, dass** die Lichtquelle, die Mittel zu Gastransporten oder beide über eine gemeinsame Halterung mit einer oder mehreren Sonden verbunden ist.

9. Verfahren zur Verwendung des Photobioreaktors nach einem der Ansprüche 1 bis 3, **dadurch gekennzeichnet, dass** die Lichtquellen eine beliebige diskontinuierliche Bewegung ausführen.

10. Verfahren nach Anspruch 9, **dadurch gekennzeichnet**, die Lichtquellen eine beliebige Bewegung mit Bewegungsumkehr ausführen.

11. Verfahren nach einem der Ansprüche 9 oder 10, **dadurch gekennzeichnet, dass** die Lichtquelle eine beliebige rotatorisch oszillierende Bewegung ausführen.

12. Verfahren nach einem der Ansprüche 9 bis 11, **dadurch gekennzeichnet, dass** Mittel zu Gastransporten eine beliebige diskontinuierliche Bewegung, eine Bewegung mit Bewegungsumkehr oder eine rotatorisch oszillierende Bewegung ausführen.

13. Verfahren nach einem der Ansprüche 9 bis 12, **dadurch gekennzeichnet, dass** die Bewegungen der Lichtquellen, der Mittel zu Gastransporten oder beider eine periodische Abfolge von Beschleunigung und Abbremsung zwischen zwei Bewegungsumkehrpunkten umfasst.

14. Verfahren nach einem der Ansprüche 1 bis 13 zur Kultivierung von Algen oder phototrophen Organismen.

15. Verwendung von Lichtleitern, Leuchtdioden oder von beiden zur Wachstumsbeförderung von phototrophen Organismen in einem Photobioreaktor.
